# DEMANDE DE BREVET EUROPEEN

(11) **EP 4 011 343 A1**
(43) Date de publication de la demande: **15.06.2022**
(21) Numéro de dépôt: 21315272.1
(22) Date de dépôt: 13.12.2021
(51) Int. Cl.: A61F 13/38

(54) **DISPOSITIF DE NETTOYAGE**

(30) Priorité: 14.12.2020 FR 2013162
(71) Demandeur: Besson, Aurélie, 59650 Villeneuve-d'Ascq (FR)
(72) Inventeur: Besson, Aurélie, 59650 Villeneuve-d'Ascq (FR)

(57) **Abrégé**

L'invention concerne un dispositif de nettoyage, constitué d'une tige comportant à au moins l'une de ses extrémités une mèche de forme allongée et constituée d'un matériau absorbant, avec l'au moins une mèche et la tige étant produites à partir d'un même matériau de base absorbant. L'invention concerne également un procédé et un appareil de fabrication dudit dispositif.

## Description

L'invention concerne un dispositif alternatif au coton-tige et destiné en particulier au nettoyage du conduit auditif externe.

Le coton-tige classique présente l'inconvénient d'être très polluant du fait de sa tige en plastique, et même si celle-ci est maintenant supposée être remplacée par une tige en papier ou un autre matériau biodégradable, il n'en est pas moins que sa fabrication nécessite deux sources de matériaux de base différents pour la tige (carton, bois, plastique) et la mèche (coton) avec les problèmes d'interface qui résultent de cette configuration, en particulier au niveau de sa production. Le coton-tige classique - en général de mèche en coton de diamètre le plus large 5 mm et de longueur 10 mm - présente en outre l'inconvénient majeur, du fait de sa mèche plus large que la tige, de pousser les sécrétions auriculaires dans le conduit auditif jusqu'à le colmater et même parfois blesser voire percer le tympan.

L'invention vise à pallier ces inconvénients et présente plusieurs avantages :
- L'utilisation d'un seul matériau de base, de préférence continu, pour la conception de la mèche et de la tige évite les problèmes d'interface et de discontinuité entre ces deux entités ;
- L'important pouvoir absorbant de la mèche permet de drainer et d'assécher rapidement le conduit auditif (en particulier après une douche), minimisant ainsi les risques d'inflammation et d'otite ;
- La mèche de longueur supérieure à celle du coton-tige classique et sa forme spécifique préférentiellement vrillée ou torsadée permettent de retirer plus efficacement les sécrétions de l'oreille externe ;
- La mise en œuvre de matériaux biodégradables et délitables permet le compostage du dispositif ou son élimination par les toilettes.

Selon l'invention, ledit dispositif comprend :
- une tige comportant à au moins l'une de ses extrémités une mèche allongée, flexible et absorbante de capacité d'absorption supérieure à 1 gramme d'eau par gramme de mèche sèche, et en outre compressible par pression de la main de l'utilisateur,
- la tige étant plus rigide et dense que la mèche,
- et l'au moins une mèche et la tige sont fabriquées à partir du même matériau de base, de préférence continu, de capacité d'absorption supérieure à 1 g/g de matériau de base.

Selon-la présente demande, la capacité d'absorption est mesurée par saturation du matériau sec par ajout de gouttes d'eau jusqu'à l'écoulement de la première goutte, indiquant la saturation. Par pesée différentielle du matériau sec et saturé en eau est calculée la capacité d'absorption du matériau.

Selon l'invention, le matériau de base représente plus de 80%, préférentiellement plus de 90% et encore plus préférentiellement plus de 95% de la masse de la mèche ; en outre, il représente plus de 40%, préférentiellement plus de 60% et plus préférentiellement plus de 70% de la masse de la tige.

Un autre avantage de la mèche absorbante et allongée est de retirer facilement les liquides et sécrétions accumulés dans le conduit auditif; en outre la mèche est exempte de partie dure en bois, plastique ou métal, que l'on retrouve dans les dispositifs commerciaux actuels et qui peuvent blesser le tympan.

La mèche peut être de forme conique, tronconique, cylindrique et/ou de section ronde ou polygonale, par exemple carrée, pentagonale ou hexagonale ; de préférence elle est de forme conique ou tronconique pour favoriser sa pénétration dans le conduit auditif.

Dans un mode particulier, la mèche comporte un mécanisme ou un produit améliorant l'extraction des sécrétions auriculaires. De préférence, ledit mécanisme réside dans la forme de la mèche qui peut être vrillée ou torsadée. Alternativement elle peut comporter des protubérances ou des creux permettant l'accrochage des sécrétions. Dans son mode alternatif mettant en œuvre un produit améliorant l'extraction des sécrétions, celui-ci peut être un produit dissolvant ou ramollissant ces dernières, ou un émollient.

Selon l'invention, la mèche est de forme allongée et de rapport longueur/diamètre moyen supérieur à 2,5 et de préférence supérieur à 3.

La mèche est de préférence 1,1 à 3 fois plus longue que celle du coton-tige classique et de diamètre moyen inférieur à ce dernier. De manière générale son diamètre moyen peut être très légèrement supérieur ou égal, mais plus préférentiellement inférieur à celui du conduit auditif externe de manière à y pénétrer facilement et en épouser la forme. De préférence son diamètre moyen est compris entre 0,5 et 5 mm, de préférence entre 1 et 4 mm. Sa longueur est de préférence supérieure à 10 mm et plus préférentiellement supérieure à 12 ou même à 15 mm. Du fait de sa texture très souple, la mèche épouse aisément la forme du conduit auditif lorsque comprimée par pression de la main sur la tige du dispositif, et permet d'en absorber le liquide et de décoller les sécrétions déposées sur ses parois. A la différence du coton-tige, la forme allongée et effilée de la mèche et son faible diamètre lui permettent de pénétrer dans le conduit sans pousser le cérumen, et l'absence de matériau dur évite à l'utilisateur de se blesser.

Le matériau de base utilisé pour la fabrication de la mèche et de la tige est de préférence biodégradable et majoritairement composé de cellulose, hémicellulose, lignine, pectine. Plus spécifiquement, il est majoritairement composé, et de préférence constitué, de fibres naturelles, telles que coton, lin, chanvre et/ou de fibres issues du bois ou de l'industrie papetière. De préférence, il est produit à partir de fibres issues de l'industrie papetière ou de produits dérivés de cette dernière.

Dans un mode préféré, le matériau de base est continu et essentiellement constitué d'un produit tissue tel que caractérisé dans la norme ISO 12625-1, par exemple le papier toilette, le papier pour mouchoir, le papier essuie-tout ou essuie-mains, ou les nappes de fibres papetières utilisées pour la fabrication desdits papiers.

La tige du dispositif, qui permet la manipulation de la mèche et sa compression, est de préférence rectiligne et de section plus large que la mèche, évitant ainsi à la tige de pénétrer trop profondément dans le canal auditif. Le matériau de base utilisé pour la confection de la tige est identique à celui de la mèche, ce qui assure une meilleure continuité et cohésion à l'ensemble et évite les problèmes d'accrochage à l'interface mèche-tige.

La tige est de préférence rendue plus rigide que la mèche par compression mécanique du matériau de base continu et/ou par ajout d'un polymère ou d'un mono/polysaccharide, de préférence biodégradable et/ou soluble dans l'eau, tel que l'alcool polyvinylique, l'amidon, la gélatine, la fécule, le saccharose ou l'agar agar. La composition papetière décrite dans le brevet EP 2379319 B1, dont le liant est préférentiellement à base d'amidon, peut par exemple être mise en œuvre pour la conception de la tige.

Le dispositif objet de l'invention est de préférence fabriqué à partir d'une bande de papier tissue de grammage compris entre 8 et 40 m²/g, de préférence compris entre 10 et 30 m²/g, et de capacité d'absorption d'eau supérieure à 1g et inférieure à 15 g par g de papier sec, de préférence supérieure à 2, 3 et même 4 g par g de papier sec. Alternativement il peut être fabriqué à partir d'une bande de fibres papetières précurseur dudit papier.

Avant la mise en forme dudit papier, la majeure partie de sa largeur - destinée à la confection de la tige - peut être imprégnée (par exemple par pulvérisation) d'une solution d'agent rigidifiant, par exemple l'alcool polyvinylique ou l'amidon, alors que le reste de sa largeur - destiné à la confection de l'au moins une mèche - est laissé vierge ou imprégné d'une quantité plus faible d'agent rigidifiant ; la bande de papier présentant un gradient de concentration en agent rigidifiant est ensuite mise en forme (par exemple par enroulement suivi d'un modelage/moulage de la mèche) et séchée.

Alternativement, le dispositif peut être produit par co-extrusion d'un cœur constitué d'un matériau fibreux absorbant précurseur de la mèche incluant le matériau de base et d'une enveloppe constituée d'un matériau fibreux précurseur de la tige plus rigide et incluant le même matériau de base, avec le cœur étant produit plus long d'au moins 10 mm que l'enveloppe, de sorte à créer une mèche dépassant du corps de la tige. Le produit ainsi extrudé est ensuite formé, par exemple pour donner une forme vrillée à la mèche, puis séché.

L'invention inclut également un procédé de fabrication du dispositif susmentionné, comprenant i) la mise à disposition du matériau de base, de préférence sous forme d'une nappe continue à base de fibres ou d'une feuille de papier tissue, ii) sa mise en forme avec densification de la partie destinée à devenir la tige, la densification étant réalisée a) par ajout audit matériau ou dépôt sur ledit matériau d'un agent rigidifiant, ou b) par compression mécanique, et la mise en forme étant de préférence réalisée par enroulement de ladite nappe ou feuille, et iii) le séchage de l'ensemble.

L'invention inclut également un appareil de fabrication du dispositif selon l'invention, comprenant i) un moyen de mise à disposition du matériau de base, de préférence sous forme d'une nappe continue à base de fibres ou d'une feuille de papier tissue, ii) un moyen de mise en forme, de préférence un moyen d'enroulement de ladite nappe ou feuille, iii) un moyen de densification de la partie destinée à devenir la tige, ledit moyen étant un moyen de compression mécanique ou un moyen d'ajout audit matériau ou un moyen de dépôt sur ledit matériau d'un agent rigidifiant, et iv) un moyen de séchage de l'ensemble.

Le moyen d'enroulement de ladite nappe ou feuille comporte en particulier deux surfaces, tels que deux rouleaux ou tapis roulants, recouvertes d'un matériau de coefficient de friction élevé, tel que le caoutchouc ou un matériau équivalent, entre lesquelles la nappe ou feuille est enroulée par friction entre les deux surfaces.

## Revendications

1. Dispositif de nettoyage du conduit auditif externe, constitué d'une tige rigide comportant à au moins l'une de ses extrémités une mèche de forme allongée et constituée d'un matériau absorbant de capacité d'absorption d'eau supérieure à 1 g/gramme de matériau absorbant sec,
**caractérisé en ce que** l'au moins une mèche et la tige sont produites à partir d'un même matériau de base absorbant de capacité d'absorption d'eau supérieure à 1g/gramme de matériau de base.

2. Dispositif selon la revendication 1, **caractérisé en ce que** le matériau de base est biodégradable et à base de cellulose, hémicellulose, lignine et/ou pectine.

3. Dispositif selon l'une quelconque des revendications précédentes, **caractérisé en ce que** le matériau de base est essentiellement constitué de fibres végétales telles que les fibres de coton, lin, jute, et/ou de fibres issues du bois ou de l'industrie papetière.

4. Dispositif selon l'une quelconque des revendications précédentes, **caractérisé en ce que** le matériau de base est essentiellement constitué de papier tissue de grammage compris entre 8 et 40 m²/g ou d'une nappe de fibres papetières précurseur dudit papier tissue.

5. Dispositif selon la revendication 1, **caractérisé en ce que** la tige rigide est obtenue par compression mécanique du matériau de base et/ou par ajout au matériau de base d'un agent rigidifiant choisi parmi les polymères et mono- ou polysaccharides biodégradables et solubles dans l'eau.

6. Dispositif selon la revendication 5, **caractérisé en ce que** l'agent rigidifiant est choisi parmi l'alcool polyvinylique, l'amidon, la gélatine, le saccharose, la fécule et/ou l'agar-agar.

7. Dispositif selon l'une des revendications précédentes, **caractérisé en ce que** la mèche est de forme torsadée ou vrillée et/ou comporte un dissolvant ou émollient, pour améliorer l'extraction des sécrétions visqueuses.

8. Dispositif selon l'une des revendications précédentes, **caractérisé en ce que** la section de l'au moins une mèche est plus faible que celle de la tige.

9. Procédé de fabrication du dispositif selon l'une des revendications 1 à 8, comprenant i) la mise à disposition du matériau de base, de préférence sous forme d'une nappe continue à base de fibres ou de papier tissue, ii) sa mise en forme avec densification de la partie destinée à devenir la tige, la densification étant réalisée par a) ajout audit matériau ou dépôt sur ledit matériau d'un agent rigidifiant, et/ou b) compression mécanique, et la mise en forme étant de préférence réalisée par enroulement de ladite nappe ou feuille, et iii) le séchage de l'ensemble.

10. Procédé selon la revendication 9, **caractérisé en ce qu'**il comprend i) la mise à disposition du matériau de base sous forme d'une nappe continue à base de fibres ou d'une feuille de papier tissue, ii) le dépôt d'une quantité d'agent rigidifiant sur au moins une partie de ladite nappe ou dudit papier, iii) l'enroulement de ladite nappe ou dudit papier et iv) le séchage de l'ensemble ainsi obtenu.

11. Appareil de fabrication du dispositif selon l'une des revendications 1 à 8, comprenant i) un moyen de mise à disposition du matériau de base, de préférence sous forme d'une nappe continue à base de fibres ou d'une feuille de papier tissue, ii) un moyen de mise en forme, de préférence un moyen d'enroulement de ladite nappe ou feuille, iii) un moyen de densification de la partie destinée à devenir la tige, ledit moyen étant un moyen de compression mécanique ou un moyen d'ajout audit matériau ou un moyen de dépôt sur ledit matériau d'un agent rigidifiant, et iv) un moyen de séchage de l'ensemble.

12. Appareil selon la revendication 11, **caractérisé en ce qu'**il comporte un moyen de mise à disposition d'une nappe continue à base de fibres ou d'une feuille de papier tissue, un moyen de dépôt d'une quantité d'agent rigidifiant sur au moins une partie de ladite nappe ou feuille, un moyen d'enroulement de ladite nappe et un moyen de séchage de l'ensemble.

13. Appareil selon la revendication 12, **caractérisé en ce que** le moyen d'enroulement de ladite nappe ou feuille comporte en particulier deux surfaces se déplaçant en sens opposé et de coefficient de friction élevé, tels que deux rouleaux ou tapis roulants dont au moins la surface est à base de caoutchouc ou d'un matériau équivalent, entre lesquelles la nappe ou feuille est enroulée par friction entre les deux surfaces.
